# EUROPÄISCHE PATENTSCHRIFT

(11) **EP 0 377 438 B1**
(45) Veröffentlichungstag und Bekanntmachung des Hinweises auf die Patenterteilung: **19.01.1994**
(21) Anmeldenummer: 90100031.5
(22) Anmeldetag: 02.01.1990
(51) Int. Cl.: C07D 305/12

(54) **Verfahren zur Verminderung des Polymergehaltes bei der Dimerisierung von Keten**
Method for the reduction of the polymer content in the dimerisation of ketene
Méthode de réduction de la concentration de polymères lors de la dimérisation de cétène

(30) Priorität: 05.01.1989 CH 30/89
(43) Veröffentlichungstag der Anmeldung: 11.07.1990
(73) Patentinhaber: LONZA AG, CH-4002 Basel (CH)
(72) Erfinder: Bergamin, Renzo, Dr., Raron, Kanton Wallis (CH); Quittmann, Wilhelm, Dr., Visp, Kanton Wallis (CH); Stoffel, Josef, Visperterminen, Kanton Wallis (CH)
(74) Vertreter: Weinhold, Peter, Dr.

(56) Entgegenhaltungen:
- US-A- 3 271 420
- E. MULLER, "Methoden der organischen Chemie" (Houben-Weyl), 4. Auflage, Band VII/4, 1968, Georg Thieme Verlag, Stuttgart (DE); Seiten 226-229#

## Beschreibung

Die vorliegende Erfindung betrifft ein Verfahren zur Senkung des Polymeranteils im Roh-Diketen, das durch katalytische Pyrolyse von Essigsäure und anschliessende Dimerisierung des gebildeten Ketens erzeugt wird.

Es ist bekannt, dass bei der Dimerisierung von Keten neben dem Hauptprodukt Diketen noch harzartige Polymere und ein Ketentrimeres gebildet werden. (D. Borrmann in Houben-Weyl, Methoden der organischen Chemie, Band 7/4, 226, 228 [1968], G.F. Pregaglia et al., Encyclop. Polymer. Sci. Techn. 8, 45 [1968]).

Die Bildung dieser Ketenpolymeren, die in einem Anteil von 8 bis 10% im Roh-Diketen vorhanden sind, bedeutet einen Wertverlust bei der Dimerisation von Keten und unerwünschtem Ballast bzw. Abfall bei der Weiterverarbeitung.

Es ist weiterhin bekannt, dass der Anteil dieser Polymeren durch Additive (Inhibitoren) wie Mineralsäuren, Acetyl- oder Benzoylchlorid, Siliciumtetrachlorid, Sulfurylchlorid (DE-PS 700 218) oder auch durch Bortrifluorid, Siliciumtetrafluorid, Zinn(II)chlorid, Chloressigsäure oder p-Toluolsulfonsäure bei der Dimerisierung in einem Lösungsmittel (DE-PS 12 40 847) gesenkt werden kann. Alle oben zitierten bekannten Inhibitoren sind entweder Protonsäuren, Metall- und Nichtmetallhalogenide oder Säurechloride.

Ein weiteres Verfahren zur Verringerung des Polymeranteils und zur Verbesserung der Lagerstabilität von Diketen verwendet elementaren Schwefel in Mengen von 0,1 bis 2 Gew.-%, bezogen auf die Diketenmenge, (DE-PS 12 68 625). Der Schwefel liegt dabei teils im Diketen gelöst, hauptsächlich jedoch suspendiert, vor.

Die bisher bekannten Inhibitoren weisen jedoch eine Reihe von Nachteilen auf. So hat sich gezeigt, dass Metallverbindungen bei der Destillation des Diketens dessen Polymerisation sogar begünstigen können, das Problem der Harzbildung also nur verlagern. Auch aus Umweltschutzgründen sind Metallverbindungen als Additive unerwünscht, da sie letztlich als Abfall beseitigt werden müssen. Säuren und Halogenverbindungen dagegen führen zu Korrosion an Apparaturen und Lagerbehältern.

Die Verwendung von elementarem Schwefel hat den Nachteil, daß vor der Verarbeitung des so stabilisierten Diketens zur Abtrennung des Schwefels eine Filtration und/oder Destillation erforderlich ist.

Aufgabe der vorliegenden Erfindung ist es, einen alternativen, metall- und halogenfreien und nicht protonsauren Inhibitor zur Anwendung zu bringen.

Erfindungsgemäss wird diese Aufgabe durch ein Verfahren dadurch gekennzeichnet, daß dem Reaktionsmedium der Dimerisierung oder dem rohen Ketengas vor der Dimerisierung Schwefeldioxid als Inhibitor in einer Konzentration von 10 bis 6000 ppm, bevorzugt 100 bis 2000 ppm, zudosiert wird gelöst.

Die Konzentrationsangabe in ppm bezieht sich dabei auf das Rohgas bzw. auf das Dimerisierungsmedium, je nachdem wo die Zudosierung erfolgt.

Der Nachteil, daß ein zusätzliches Lösungsmittel wie in der DE-PS 12 40 847 verwendet werden muß, tritt bei der Verwendung von Schwefeldioxid als Inhibitor nicht auf.

Die Herstellung von Keten und deren Weiterverarbeitung zu Diketen sind z.B. in "Ullmanns Encyklopädie der technischen Chemie" (4.Aufl., Band 14, Seiten 181 ff) beschrieben. Die Ketenherstellung durch katalytische Pyrolyse von Essigsäure wird bevorzugt.

Das Verfahren der Inhibierung eignet sich sowohl für die batchweise (Beispiel 3) als auch für die kontinuierliche (Beispiele 1 und 2) Dimerisierung von Keten.

Durch die Zudosierung des oben genannten Inhibitors werden anionische Polymerisationsketten abgebrochen bzw. Spuren polymerisationsauslösender Basen desaktiviert.

Das Verfahren kann im Rührkessel mit Gaseinleitungsrohr oder aber in der üblicherweise zum Verdichten des rohen Ketengases verwendeten Flüssigkeitsringpumpe oder in Gegenstromabsorbern ausgeführt werden. Bei kontinuierlicher Arbeitsweise hat es sich als vorteilhaft erwiesen, die zugeführte Schwefeldioxidmenge anhand des analytisch bestimmten Schwefelgehaltes im Diketenprodukt zu regeln.

Als Polymeranteil wird analytisch die Menge des Diketenproduktes definiert, die bei der Vakuumverdampfung (1,33Pa (0,01Torr)) bei Raumtemperatur nach 2 Stunden als Rückstand verbleibt.

### Beispiel l (kontinuierliches Verfahren)

In die Saugseite der Flüssigkeitsring-Vakuumpumpe, die zum Verdichten des durch katalytische Pyrolyse von Essigsäure erhaltenen rohen Ketengases dient, wurden bis zu 550 ppm Schwefeldioxid (bezogen auf die Rohgasmenge) eingespeist. Als Pumpenringflüssigkeit wurde Rein-Diketen verwendet. Bei Reaktionstemperaturen von 20 bis 32°C resultierte eine Verminderung des Polymeranteils von 1,5 bis 9% gegenüber einer Versuchsdurchführung ohne Schwefeldioxid (Tabelle 1).

**Tabelle 1**

| SO₂-Konzentration [ppm] | Polymeranteil [%] | Dimerisierungsmedium | Reaktionstemperatur (Mittelwert) [°C] |
|---|---|---|---|
| 0 | 12,5 | Reindiketen | 24,4 |
| 150 - 250 | 10,5 - 11,0 | Reindiketen | 27,5 |
| 450 - 550 | 3,5 - 3,8 | Reindiketen | 27,5 |

### Beispiel 2 (kontinuierliches Verfahren)

In die gleiche Apparatur, wie in Beispiel 1 beschrieben, wurde Schwefeldioxid in solcher Menge eingeleitet, dass die stationäre Schwefelkonzentration im Dimerisierungsmedium bis zu 2850 ppm S betrug. Die beobachtete Senkung des Polymeranteils betrug 3,2 bis 6,4% (Tabelle 2).

**Tabelle 2**

| Schwefelkonz. i. Produkt [ppm] | Polymeranteil [%] | Dimerisierungsmedium | Reaktionstemperatur (Mittelwert) [°C] |
|---|---|---|---|
| 0 | 13,0 | Rohdiketen | 23 |
| 530 | 9,8 | Rohdiketen | 23 |
| 790 | 6,6 | Rohdiketen | 23 |
| 900 | 8,1 | Rohdiketen | 23 |
| 2850 | 9,5 | Rohdiketen | 23 |

### Beispiel 3 (Batch-Verfahren)

In ein 1,5 l Glasrührwerk mit Doppelmantel zur Kühlung wurden bei 12 ± 2°C in 1 Liter Diketen mit SO₂ vermischtes rohes Ketengas zur Dimerisierung eingeleitet. Am Ende des Versuches wurde der Polymeranteil wie oben angegeben bestimmt und auf das produzierte Diketen umgerechnet (Tabelle 3).

**Tabelle 3**

| SO₂-Konzentraion [ppm] | Polymeranteil [%] | Dimerisierungsmedium |
|---|---|---|
| 0 | 14,5 - 15,3 | Reindiketen |
| 160 | 7,6 - 8,3 | Reindiketen |
| 84 | 8,7 | Reindiketen |
| 0 | 11,6 | Rohdiketen |
| 1940 | 10,2 | Rohdiketen |
| 750 | 9,0 | 2/3 Roh- + 1/3 destilliertes Diketen |

## Patentansprüche

1. Verfahren zur Verminderung des Polymeranteils bei der Dimerisierung von Keten, dadurch gekennzeichnet, daß dem Reaktionsmedium der Dimerisierung oder dem rohen Ketengas vor der Dimerisierung Schwefeldioxid als Inhibitor in einer Konzentration von 10 bis 6000 ppm zudosiert wird.

2. Verfahren nach Anspruch 1, dadurch gekennzeichnet, daß das Schwefeldioxid in einer Konzentration von 100 bis 2000 ppm zudosiert wird.

## Claims

1. A process for the reduction of the polymer proportion when dimerizing keten, characterized in that before the dimerization sulfur dioxide is added as inhibitor in a concentration of 10 to 6000 ppm to the reaction medium of the dimerization or the crude keten gas.

2. A process according to claim 1, characterized in that the sulfur dioxide is added in a concentration of 100 to 2000 ppm.

## Revendications

1. Procédé pour réduire la proportion de polymère lors de la dimérisation du cétène, caractérisé en ce que le milieu réactionnel de la dimérisation ou le cétène gazeux brut est additionné de manière dosée avant la dimérisation de dioxyde de soufre en tant qu'inhibiteur en une concentration de 10 à 6000 ppm.

2. Procédé selon la revendication 1, caractérisé en ce que le dioxyde de soufre est ajouté de manière dosée en une concentration de 100 à 2000 ppm.
